# EUROPEAN PATENT APPLICATION

(11) **EP 3 469 998 A1**
(43) Date of publication of application: **17.04.2019**
(21) Application number: 17809648.3
(22) Date of filing: 27.05.2017
(51) Int. Cl.: A61B 17/32, A61B 17/16

(54) **ULTRASOUND BONE CUTTING TOOL**

(30) Priority: 08.06.2016 CN 201620555006 U
(71) Applicant: Jiangsu SMTP Technology Co., Ltd., Zhangjiagang, Jiangsu 215634 (CN)
(72) Inventor: SUN, Yu, Zhangjiagang Jiangsu 215634 (CN); ZHAN, Songtao, Zhangjiagang Jiangsu 215634 (CN); CAO, Qun, Zhangjiagang Jiangsu 215634 (CN)
(74) Representative: Balder IP Law, S.L.
(86) International application number: PCT/CN2017/086366
(87) International publication number: WO 2017/211209

(57) **Abstract**

Disclosed is a tool bit for an ultrasonic osteotome comprising a grinding portion at a front end of the tool bit for an ultrasonic osteotome, and an arbor with one end connected to the grinding portion and the other end connected with a transducer for an ultrasonic osteotome. The grinding portion has a tapered shape dwindling from a rear end toward a front end thereof and is connected with the arbor at the rear end thereof. A plurality of grinding grooves are provided on a bottom surface and side surfaces of the grinding portion, and a front surface of the grinding portion is a smooth surface. The tool bit for an ultrasonic osteotome has a tapered shape, which is small in structure, simple and flexible for operation, and convenient in use. When the tool bit for an ultrasonic osteotome of the present disclosure is used for a surgery, the ultrasonic energy is concentrated at a tip end, which greatly improves operation efficiency, and a surgical window has a large field of view, which is convenient for a doctor to observe operation condition while performing the operation, avoiding a surgical blind zone and reducing risk of surgery. Postoperative wound surfaces have good contact therebetween, which is advantageous for bone healing and reduces the risk of complications. The patient has less pain and quick recovery.

## Description

### TECHNICAL FIELD

The present disclosure relates to the field of medical instruments and devices, in particular to a scalpel, and more particularly to a tool bit for an ultrasonic osteotome.

### BACKGROUND OF THE INVENTION

In modern society, with the development of medical technology, orthopedic surgery shows a trend of diversity. Accordingly, when performing a surgery, it is necessary to use different tool bits for a scalpel to perform cutting, grinding, scraping, clamping and other operations on an affected area in a patient according to different orthopedic conditions of disease.

In view of the special construction of bone structure, along with the continuously development of ultrasound technology in recent years, an ultrasonic osteotome has gradually become a main tool for modern orthopedic surgery. An ultrasonic osteotome employs high-intensity focused ultrasound technique that converts electrical energy to mechanical energy through a transducer, and evaporates water in the contacted tissue cells and breaks protein hydrogen bonds through high-frequency ultrasound concussion, so as to completely destroy bone tissues to be cut in the surgery. Since the high-intensity focused ultrasound only has a destructive effect on bone tissues of a specific hardness and has a characteristic of cutting hard things rather than soft things, it is particularly suitable for spinal surgery in which the peripheral structure is a bone structure and the middle structure is a soft tissue as a spinal cord. Surgery with an ultrasonic osteotome can effectively prevent the occurrence of medical accidents during the operation such as an injury to the spinal cord due to excessive force, thereby improving the safety of the operation.

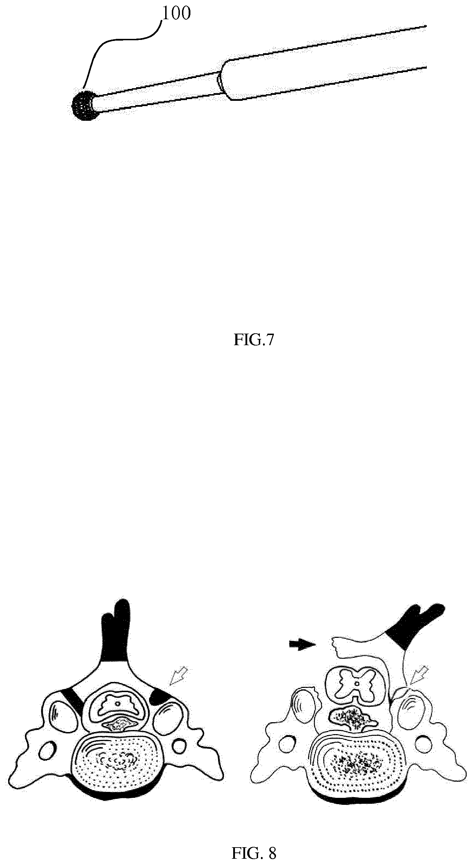

In a surgical operation for grinding a bone to opening a notch in a bone, typically, a tool bit for an ultrasonic osteotome having a square or round head is used, as shown in Fig. 7. When these two types of tool bits for an ultrasonic osteotome are used for grinding and removing bone, the amount of bone removed is large, the speed of operation is slow, and in addition, when performing a spinal open-door shaft side grinding, due to the specific shape of the tool bit for an ultrasonic osteotome used in the prior art, a square or round pit will be incurred by grinding. The existence of the pit makes grinded bone surfaces on opposite sides not able to contact with each other tightly when the two surfaces are placed against each other, and there will be a cavity shaped like the tool bit for an ultrasonic osteotome between the opposite bone surfaces, as shown in Fig. 8. In this way, a patient's healing time is greatly prolonged, and because of the presence of the cavity, the risk of postoperative complications is greatly increased.

### SUMMARY OF THE INVENTION

In order to solve the above problems of the prior art, the present disclosure provides a tool bit for an ultrasonic osteotome comprising a grinding portion at a front end of the tool bit for an ultrasonic osteotome, and an arbor with one end connected to the grinding portion and the other end connected with a transducer for an ultrasonic osteotome. The grinding portion has a tapered shape dwindling from a rear end toward a front end thereof and is connected with the arbor at the rear end thereof. A plurality of grinding grooves are provided on a bottom surface and side surfaces of the grinding portion, and a front surface of the grinding portion is a smooth surface.

The tool bit for an ultrasonic osteotome of the present disclosure has a small area at a front end, which facilitates focusing energy of the transducer for an ultrasonic osteotome to the front end. Therefore, the tool bit for an ultrasonic osteotome of the present disclosure provides a quick grinding speed and a high working efficiency. In addition, the tool bit for an ultrasonic osteotome of the present disclosure has a large area at a rear end, which can provide a medical staff member with a good view for a surgical operation, avoiding a blind zone operation, reducing risk for an operation, and improving safety of an operation. Meanwhile, in a single open-door surgery of the spine, due to the special shape of small front end and large rear end of the tool bit for an ultrasonic osteotome of the present disclosure, a door shaft-side bone surface which is ground by the tool bit for an ultrasonic osteotome of the present disclosure can be sufficiently closed, thereby promoting growth and fusion of the bone, further shortening a recovery time for a patient and reducing patient pain.

According to an embodiment of the tool bit for an ultrasonic osteotome of the present invention, the grinding grooves of the tool bit for an ultrasonic osteotome of the present invention each have a certain width, and the grinding grooves are provided with reverse fine blades, which are arranged to facilitate scraping a bone while grinding the bone.

According to an embodiment of the tool bit for an ultrasonic osteotome of the present invention, a shape of the grinding portion is a trapezoidal prism having a large rear end and a small front end, and an upper and a lower surfaces are parallel to each other and parallel to an axis of the tool bit for an ultrasonic osteotome. Alternatively, a shape of the grinding portion is a triangular pyramid having a large rear end and a pointed front end, and an upper surface and a lower surface are parallel to each other and parallel to an axis of the tool bit for an ultrasonic osteotome. A medical staff member can choose from tool bits of different shapes as desired in an operation.

According to an embodiment of the tool bit for an ultrasonic osteotome of the present invention, the grinding grooves are transverse grooves perpendicular to the axis of the tool bit for an ultrasonic osteotome.

According to an embodiment of the tool bit for an ultrasonic osteotome of the present invention, the grinding grooves are skewed grooves at an angle with the axis of the tool bit for an ultrasonic osteotome.

According to an embodiment of the tool bit for an ultrasonic osteotome of the present invention, the grinding grooves are knurled teeth.

According to an embodiment of the tool bit for an ultrasonic osteotome of the present invention, the smooth surface at the front end of the grinding portion is a flat surface.

According to an embodiment of the tool bit for an ultrasonic osteotome of the present invention, the smooth surface at the front end of the grinding portion is a convex curved surface.

According to an embodiment of the tool bit for an ultrasonic osteotome of the present invention, a bottom surface of the grinding portion is inclined to an axis of the tool bit for an ultrasonic osteotome and intersects the axis of the tool bit for an ultrasonic osteotome at the front end of the grinding portion. A medical staff member can choose a tool bit for an ultrasonic osteotome owning a plane with a certain angle for an operation as desired in use, so as to make the operation more convenient and handy.

Compared with the prior art, the tool bit for an ultrasonic osteotome according to the embodiments of the invention has the following advantages: the tool bit for an ultrasonic osteotome has a tapered shape, which is small in structure, simple and flexible for operation, and convenient in use. When the tool bit for an ultrasonic osteotome of the present disclosure is used for a surgery, the ultrasonic energy is concentrated at a tip end, which greatly improves operation efficiency, and a surgical window has a large field of view, which is convenient for a doctor to observe operation condition while performing the operation, avoiding a surgical blind spot and reducing risk of surgery. Postoperative wound surfaces have good contact therebetween, which is advantageous for bone healing and reduces the risk of complications. The patient has less pain and quick recovery.

### BRIEF DESCRIPTION OF THE DRAWINGS

In order to more clearly illustrate the specific embodiments of the present invention or the technical solutions in the prior art, the drawings used in the specific embodiments or the description of the prior art will be briefly described below. Apparently, the drawings illustrated show only some embodiments of the present invention, and those skilled in the art can obtain other drawings based on these drawings without creative work.
Fig. 1 is a schematic perspective view of a first embodiment of a tool bit for an ultrasonic osteotome of the present invention;
Fig. 2 is a schematic front elevational view showing the first embodiment of the tool bit for an ultrasonic osteotome of the present invention;
Fig. 3 is a schematic bottom plan view showing a first embodiment of the tool bit for an ultrasonic osteotome of the present invention;
Fig. 4 is a schematic view showing a grinding portion of a second embodiment of a tool bit for an ultrasonic osteotome of the present invention;
Fig. 5 is a schematic view showing a grinding portion of a third embodiment of a tool bit for an ultrasonic osteotome of the present invention;
Fig. 6 is a schematic front elevational view showing a fourth embodiment of a tool bit for an ultrasonic osteotome of the present invention;
Fig. 7 is a schematic perspective view of a grinding portion of a tool bit for an ultrasonic osteotome in the prior art;
Fig. 8 is a schematic view showing a state of bone tissues after an orthopedic single door operation using a grinding portion of a tool bit for an ultrasonic osteotome in the prior art;
Fig. 9 is a schematic view showing a state of bone tissues after an orthopedic single door surgery using a grinding portion of a tool bit for an ultrasonic osteotome of the present invention.

### List of Reference Numerals:

1,100 grinding portion; 2 arbor; 3 grinding groove; 4 smooth surface; 5 fine blade; 6 thread; 7 clamping surface

### DETAILED DESCRIPTION OF THE INVENTION

Exemplary embodiments of the present disclosure will be described hereinafter clearly and completely with reference to the attached drawings. Apparently, the embodiments described herein are only portions of embodiments of the disclosure, rather than all embodiments of the disclosure. It is intended that all other embodiments obtained by those skilled in the art according to the disclosed embodiments without inventive labor are within the scope of the present invention.

In the description of the present disclosure, it is to be noted that the terms of "center" , "upper" , "lower" , "left" , "right" , "vertical" , "horizontal" , "internal" , " external" and the like simply indicate orientational or positional relationship based on the accompanying drawings and are used only for the purpose of facilitating and simplifying the description of the invention, rather than specifying or implying that any device or elements indicated must have a certain orientation, constitute with a certain orientation, or operate in a certain orientation. Therefore, these terms will not be interpreted as limiting the present invention. Further, the terms of "first" , "second" and "third" are only used for description purpose, rather than being interpreted as specifying or implying relative importance.

In the description of the present disclosure, it is to be noted that, unless otherwise specified or defined clearly, the term of "attach, "connect to" , "connect with" , "couple" and the like should be interpreted broadly. For example, they may refer to fixed connection, or detachable connection, or integral connection; they may refer to mechanical connection, or electrical connection; they may refer to direct connection, or indirect connection through an intermediate agent, or internal communication between two components. For those skilled in the art, the specific meaning of these terms in the present disclosure may be understood in combination with specific situations or contexts.

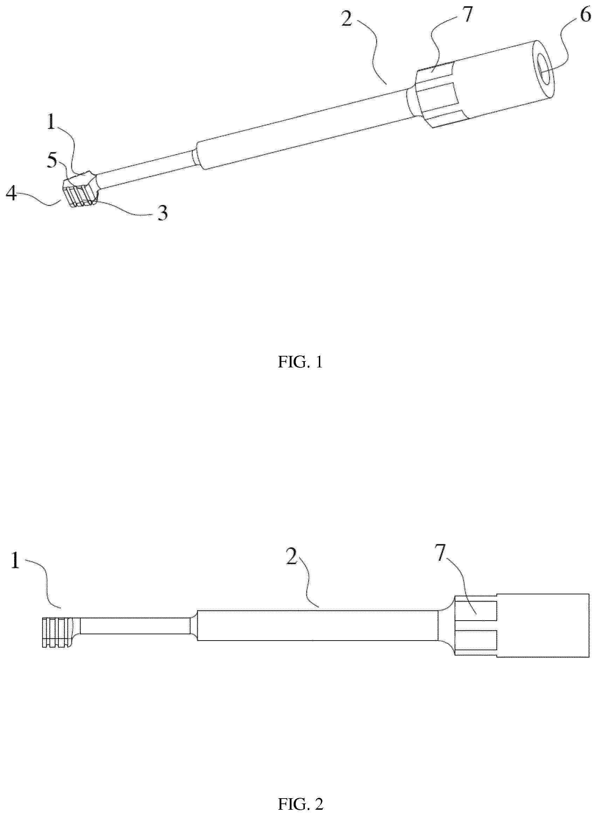

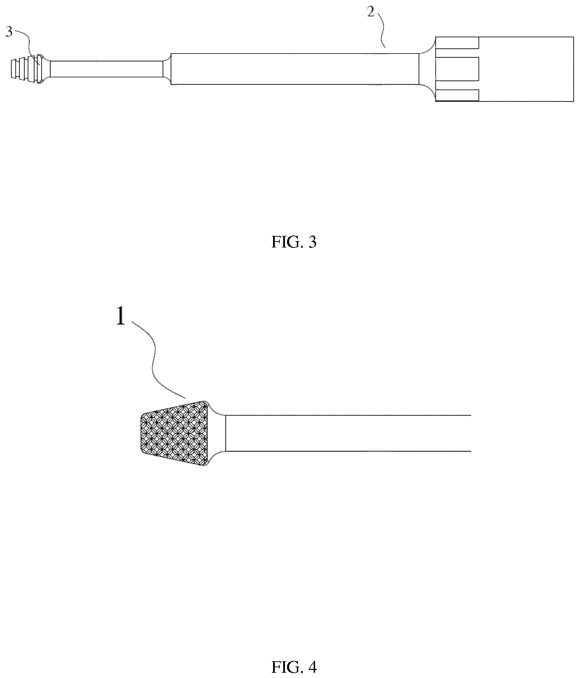

Fig. 1 is a schematic perspective view of a first embodiment of a tool bit for an ultrasonic osteotome of the present invention. Fig. 2 is a schematic front elevational view showing the first embodiment of the tool bit for an ultrasonic osteotome of the present invention. Fig. 3 is a schematic bottom plan view showing a first embodiment of the tool bit for an ultrasonic osteotome of the present invention. As shown in Figs. 1-3, the tool bit for an ultrasonic osteotome according to the first embodiment of the present invention comprises a grinding portion 1 at a front end of the tool bit for an ultrasonic osteotome, and an arbor 2 with one end connected to the grinding portion 1 and the other end connected with a transducer for an ultrasonic osteotome. The arbor 2 may have a cylindrical shape, and a thread 6 for connecting with the transducer for an ultrasonic osteotome is provided at a tail end thereof. The thread 6 may be internally threaded or externally threaded according to the requirement for connection. A plurality of clamping faces 7 for clamping may also be provided on a cylindrical surface of the arbor 2. In use, an operator may connect the arbor 2 to the transducer for the ultrasonic osteotome, and then clamp the clamping faces 7 with a tool such as a wrench, and rotate the tool bit for an ultrasonic osteotome in a tightening direction, thereby the tool bit for an ultrasonic osteotome can be firmly fixed to the transducer for an ultrasonic osteotome to avoid a danger of falling off of the tool bit for an ultrasonic osteotome during a surgery.

As shown in Figs. 1 and 3, the grinding portion 1 has a tapered shape dwindling from a rear end toward a front end thereof, such as a trapezoid. The grinding portion 1 is connected to the arbor 2 at the rear end thereof, and a plurality of grinding grooves 3 for grinding a bone are formed on a bottom surface and side surfaces of the grinding portion 1, and a front surface of the grinding portion 1 is a smooth surface 4.

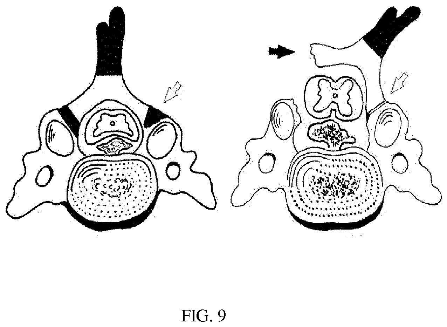

The tool bit for an ultrasonic osteotome of the present embodiment has a small area at a front end, which facilitates focusing energy of the transducer for an ultrasonic osteotome to the front end. Therefore, the tool bit for an ultrasonic osteotome of the present embodiment provides a quick grinding speed and a high working efficiency. In addition, the tool bit for an ultrasonic osteotome of the present embodiment has a large area at a rear end, which can provide a medical staff member with a good view for a surgical operation, avoiding a blind zone operation, reducing risk for an operation, and improving safety of an operation. Meanwhile, as shown in Fig. 9, when the tool bit for an ultrasonic osteotome of the present embodiment are used to perform a single open-door surgery on a spine, due to the special shape of small front end and large rear end of the tool bit, a door shaft-side bone surface which is ground by the tool bit can be sufficiently closed, thereby promoting growth and fusion of the bone, further shortening a recovery time for a patient and reducing patient pain.

The grinding grooves 3 of the tool bit for an ultrasonic osteotome of the present embodiment have a certain width, and the grinding grooves 3 are provided with reverse fine blades 5, which are arranged to facilitate bone scraping while grinding the bone. When bone scraping and grinding are performed at the same time, the efficiency of surgery can be greatly improved.

Fig. 4 is a schematic view of a grinding portion of a second embodiment of the tool bit for an ultrasonic osteotome of the present invention. In Fig. 4, a shape of the grinding portion 1 of the tool bit for an ultrasonic osteotome may be a trapezoidal prism which has a large rear end and a small front end, and the upper and lower surfaces are parallel to each other and parallel to an axis of the tool bit for an ultrasonic osteotome. Alternatively, a shape of the grinding portion 1 of the tool bit for an ultrasonic osteotome may be a trapezoidal cylinder, which has a large rear end and a small front end and has a trapezoidal shape in a section along the axis of the tool bit for an ultrasonic osteotome.

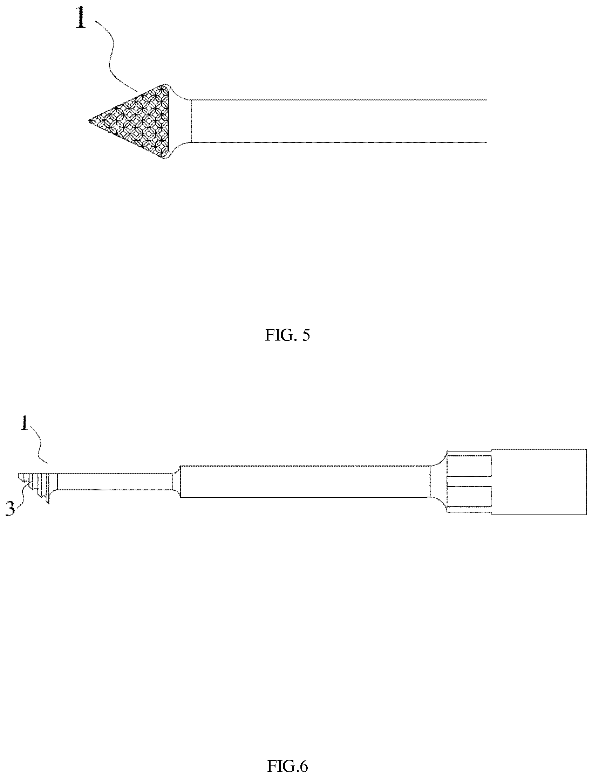

Fig. 5 is a schematic view showing a grinding portion of a third embodiment of the tool bit for an ultrasonic osteotome of the present invention. In Fig. 5, a shape of the grinding portion 1 of the tool bit for an ultrasonic osteotome may be a pyramid, which has a large rear end and a pointed front end, and the upper surface and lower surface thereof are parallel to each other and parallel to an axis of the tool bit for an ultrasonic osteotome. Alternatively, the shape of the grinding portion 1 of the tool bit for an ultrasonic osteotome may also be a pyramid or a cone having a large rear end and a pointed front end. A medical staff member can use different shapes of tool bits for an ultrasonic osteotome according to the needs of an operation.

In the tool bit for an ultrasonic osteotome shown in Fig. 3 to 5, the grinding grooves 3 may be transverse grooves perpendicular to an axis of the tool bit for an ultrasonic osteotome, or skewed grooves formed at an angle with the axis of the tool bit for an ultrasonic osteotome, or knurled teeth. The transverse grooves, the skewed grooves, or the knurled teeth are all shapes that can be obtained by conventional machining. For knurled teeth, a special knurling knife can be used for machining. Therefore, the tool bit for an ultrasonic osteotome of the present invention is easy to manufacture and has a low manufacture cost.

In the tool bit for an ultrasonic osteotome of the present invention, the smooth surface 4 at the front end of the grinding portion 1 may be a flat surface or a convex curved surface. The smooth surface of this structure can protect a patient's soft tissues from unnecessary hurt resulted from instrumentation of surgery.

Fig. 6 is a schematic front elevational view showing a fourth embodiment of the tool bit for an ultrasonic osteotome of the present invention. In Fig. 6, a bottom surface of the grinding portion 1 is inclined to an axis of the tool bit for an ultrasonic osteotome and intersects the axis of the tool bit for an ultrasonic osteotome at a front end of the grinding portion. As viewed from a side, a shape of the grinding portion is a triangular. The triangular shape provides a bevel allowing a medical staff member to select an optimal angle for grinding according to the needs of an operation, so that the operation can be performed more smoothly, which can improve operation efficiency, reduce fatigue of the doctor in operation and reduce the risk of surgery.

The tool bit for an ultrasonic osteotome of the disclosure adopts rounded transition at all corners, which avoids any possible hurt on soft body tissues by scratching. Compared with the prior art, embodiments of the invention have the following advantages: the tool bit for an ultrasonic osteotome has a tapered shape, which is small in structure, simple and flexible for operation, and convenient in use. When the tool bit for an ultrasonic osteotome of the present disclosure is used for a surgery, the ultrasonic energy is concentrated at a tip end, which greatly improves operation efficiency, and a surgical working face has a large field of view, which is convenient for a doctor to observe operation condition while performing the operation, avoiding a surgical blind spot and reducing risk of surgery. As shown in Fig.9, postoperative wound surfaces have good contact therebetween, which is advantageous for bone healing and reduces the risk of complications. The patient has less pain and quick recovery.

It should be noted that the above embodiments are only used to describe the solutions of the present invention, rather than limiting the present invention. The protection scope of the invention is defined by the claims. Although detailed descriptions of the invention are made with reference to the above embodiments, it would be appreciated by those skilled in the art that various changes or modifications to the above embodiments may be made or equivalent substitutions to portion of or all features in those embodiments may be made. Such changes, modifications or substitutions will not make the spirit of the relevant solutions depart from the scope of the present invention.

## Claims

1. A tool bit for an ultrasonic osteotome comprising:
a grinding portion located at a front end of the tool bit for an ultrasonic osteotome, and an arbor with one end connected to the grinding portion and the other end connected with a transducer for an ultrasonic osteotome, wherein
the grinding portion has a tapered shape dwindling from a rear end toward a front end thereof and is connected with the arbor at the rear end thereof, and
a plurality of grinding grooves are provided on a bottom surface and side surfaces of the grinding portion, and a front surface of the grinding portion is a smooth surface.

2. The tool bit for an ultrasonic osteotome according to claim 1, wherein
the grinding grooves each have a certain width, and the grinding grooves are provided with reverse fine blades thereon.

3. The tool bit for an ultrasonic osteotome according to claim 1, wherein
a shape of the grinding portion is a trapezoidal prism having a large rear end and a small front end, and an upper surface and a lower surface thereof are parallel to each other and parallel to an axis of the tool bit for an ultrasonic osteotome.

4. The tool bit for an ultrasonic osteotome according to claim 1, wherein
a shape of the grinding portion is a triangular pyramid having a large rear end and a pointed front end, and an upper surface and a lower surface thereof are parallel to each other and parallel to an axis of the tool bit for an ultrasonic osteotome.

5. The tool bit for an ultrasonic osteotome according to any one of claims 1 to 4, wherein
the grinding grooves are transverse grooves perpendicular to an axis of the tool bit for an ultrasonic osteotome.

6. The tool bit for an ultrasonic osteotome according to any one of claims 1 to 4, wherein
the grinding grooves are skewed grooves formed at an angle with an axis of the tool bit for an ultrasonic osteotome.

7. The tool bit for an ultrasonic osteotome according to any one of claims 1 to 4, wherein
the grinding grooves are knurled teeth.

8. The tool bit for an ultrasonic osteotome according to any one of claims 1 to 4, wherein
the smooth surface is a flat surface.

9. The tool bit for an ultrasonic osteotome according to any one of claims 1 to 4, wherein
the smooth surface is a convex curved surface.

10. The tool bit for an ultrasonic osteotome according to claim 1 or 2, wherein
a bottom surface of the grinding portion is inclined to an axis of the tool bit for an ultrasonic osteotome and intersects the axis of the tool bit for an ultrasonic osteotome at the front end of the grinding portion.
